(19) Europäisches Patentamt
European Patent Office
Office européen des brevets

(11) **EP 1 255 103 B1**

(12) **EUROPÄISCHE PATENTSCHRIFT**

(45) Veröffentlichungstag und Bekanntmachung des Hinweises auf die Patenterteilung:
**13.02.2008 Patentblatt 2008/07**

(51) Int Cl.:
*G01N 9/36* (2006.01)  *G01N 33/34* (2006.01)
*D21G 9/00* (2006.01)

(21) Anmeldenummer: **02005683.4**

(22) Anmeldetag: **13.03.2002**

(54) **Verfahren und Vorrichtung zum Kalibrieren von Stoffdichtesensoren**

Method and apparatus for calibrating density sensors

Méthode et appareil pour calibrer des senseurs de masse spécifique

(84) Benannte Vertragsstaaten:
**AT DE FI IT SE**

(30) Priorität: **04.05.2001 DE 10121775**

(43) Veröffentlichungstag der Anmeldung:
**06.11.2002 Patentblatt 2002/45**

(73) Patentinhaber: **Voith Patent GmbH**
**89522 Heidenheim (DE)**

(72) Erfinder: **Schwarz, Michael, Dr.**
**89522 Heidenheim (DE)**

(56) Entgegenhaltungen:
**EP-A- 0 785 305**   **DE-A- 10 043 142**
**DE-A- 19 913 926**   **US-A- 5 099 118**

Bemerkungen:
Die Akte enthält technische Angaben, die nach dem Eingang der Anmeldung eingereicht wurden und die nicht in dieser Patentschrift enthalten sind.

EP 1 255 103 B1

## Beschreibung

Die Erfindung betrifft ein Verfahren sowie eine Vorrichtung zum Kalibrieren von Stoffdichtesensoren, die bei der Herstellung einer Faserstoffbahn, insbesondere Papier- oder Kartonbahn, dazu dienen, für den Herstellungsprozeß benötigte Stoffdichten von Faserstoffsuspensionen zu messen.

Im Stoffzuführsystem, d.h. im konstanten Teil einer der Papierherstellung dienenden Anlage ist die Messung der Stoffdichte mit die wichtigste Kenngröße zur Prozeßführung. Entsprechend ist es bekannt, Konzentrationen von Faserstoffsuspensionen, d.h. sowohl von Suspensionen mit hoher Stoffdichte (Konzentration g/l von Faserstoff) als auch von Suspensionen mit kleiner Stoffdichte (z.B. Siebwasser I, Siebwasser II), zu messen und die Sensoren entsprechend anzuordnen. Die Messung in einer Suspension ist jedoch vom Meßprinzip her schwierig und kann durch Änderungen der Stoffzusammensetzung oder der Füllstoffart verfälscht werden. Von Nachteil ist insbesondere, daß die Sensoren offline, d.h. im ausgebauten Zustand, oder durch stichprobenartiges Überprüfen anhand einer besseren Stoffdichte im Labor kontrolliert und nachkalibriert werden müssen, was entsprechend zeit- und kostenintensiv ist. Durch die Kalibrierung wird der entsprechende Zusammenhang zwischen dem von der Meßeinheit ausgegebenen Stoffdichte-Meßwert und der tatsächlichen Stoffdichte hergestellt.

In dem Dokument DE 100 43 142 A1 werden ein Verfahren und eine Vorrichtung zur Regelung eines stoffdichtegeregelten Stoffauflaufs einer Papier- oder Kartonmaschine offenbart, wobei ein vorzugsweise am Ende der Papier- oder Kartonmaschine angeordneter Flächengewichtssensor vorgesehen ist, der sich zur Kalibirierung eines Berechnungsmodells eignet, welches zur Modellierung verschiedener Prozessparameter dient.

Ziel der Erfindung ist es, ein verbessertes Verfahren sowie eine verbesserte Vorrichtung der eingangs genannten Art anzugeben, mit denen die betreffenden Stoffdichtesensoren auf einfachere und wirschaftlichere Weise kalibriert werden können.

Diese Aufgabe wird erfindungsgemäß gelöst durch ein Verfahren zum Kalibrieren von Stoffdichtesensoren, die bei der Herstellung einer Faserstoffbahn, insbesondere Papier- oder Kartonbahn, dazu dienen, für den Herstellungsprozess benötigte Stoffdichten von Faserstoffsuspensionen zu messen, wobei auf ein und demselben Messprinzip basierende Stoffdichtesensoren verwendet und diese Stoffdichtesensoren online und automatisch kalibriert werden, indem zur Aufstellung wenigstens einer Massenbilanz zumindest ein Teil der in eine Bilanzhülle eintretenden und aus dieser austretenden Volumen- und / oder Mengenströme gemessen werden und indem ein Bilanzierungsmodell durch mathematische Verknüpfung aufgestellt wird, bei der die wenigstens eine Massenbilanz und die Sensorkennlinien der Stoffdichtesensoren Berücksichtigung finden, um aus den erhaltenen Stoffdichtemesswerten die tatsächlichen Stoffdichtewerte zu ermitteln.

Aufgrund dieser Ausgestaltung ist es möglich, die Stoffdichtesensoren online und automatisiert zu kalibrieren. Die gemessenen Stoffdichten können nunmehr quasi kontinuierlich überprüft und korrigiert werden, wodurch der Papierherstellungsprozeß stabiler und effizienter wird, da die damit zusammenhängenden Betriebs- bzw. Qualitätsparameter, wie insbesondere Stoffdichten, Retention, Längsprofile des Flächengewichts, Formation, usw., weniger Schwankungen unterliegen und daher nahezu konstant sind. Im Ergebnis wird die Produktqualität entsprechend erhöht. Mit der automatischen Kalibrierung der Stoffdichtesensoren oder -transmitter ergibt sich eine präzisere Prozeßregelung sowie eine Kosteneinsparung.

Gemäß einer bevorzugten Ausgestaltung des erfindungsgemäßen Verfahrens wird überdies ein Stoffdichtesensor verwendet, der einen mit dem betreffenden tatsächlichen Stoffdichtewert übereinstimmenden Stoffdichtemeßwert liefert, wobei dieser direkt verwendbare Stoffdichtemeßwert bei der Aufstellung der Massenbilanz mit herangezogen wird.

Die zu kalibrierenden Stoffdichtesensoren können insbesondere im konstanten Teil, d.h. in der Stoffzuführung, der Herstellungsanlage eingesetzt sein.

Die zu kalibrierenden Stoffdichtesensoren können beispielsweise lineare Sensorkennlinien besitzen.

Die zu kalibrierenden Stoffdichtesensoren sind vorzugsweise Leitungen zugeordnet, in denen Faserstoff mit derselben Stoffcharakteristik bzw. Stoff mit derselben Stoffcharakteristik fließt.

Gemäß einer zweckmäßigen Ausgestaltung des erfindungsgemäßen Verfahrens sind die zu kalibrierenden Stoffdichtesensoren jeweils einer Dickstoff führenden Leitung zugeordnet.

So kann beispielsweise eine Massenbilanz für einen Teilabschnitt der Herstellungsanlage aufgestellt werden, dem Dickstoff und Verdünnungswasser zugeführt und aus dem verdünnter Dickstoff abgeführt wird, wobei die zu kalibrierenden Stoffdichtesensoren zur Messung der Stoffdichten des zugeführten unverdünnten Dickstoffes und des abgeführten verdünnten Dickstoffes vorgesehen sind.

In bestimmten Fällen ist es auch von Vorteil, wenn eine Massenbilanz aufgestellt wird, in die die gemessene Flächenmasse nach der Papiermaschine und/oder in der Trockenpartie und/oder nach der Presse und/oder der gemessene Trockengehalt nach der Presse mit einbezogen wird.

Wird das gesamte System des Naßprozesses (wet end process) zusammen als geschlossenes Modell in der betreffenden Regelungsarchitektur abgebildet, so kann der genau arbeitende Sensor beispielsweise am Ende der Papiermaschine als Kalibriersensor online oder periodisch die Suspensionssensoren im konstanten Teil prüfen und gegebenenfalls nachstellen. Dies gilt in gleichem Maße sowohl für die Gesamtfeststoffdichte als auch für die Füllstoffkonzentration, da beide Werte sowohl mittels der Suspensionssensoren als auch mit dem traversierenden Scanner erfaßt werden können.

Die erfindungsgemäße Aufgabe wird gelöst durch eine Vorrichtung zum Kalibrieren von Stoffdichtesensoren, die bei der Herstellung einer Faserstoffbahn, insbesondere Papier- oder Kartonbahn, dazu dienen, für den Herstellungsprozeß benötigte Stoffdichten von Faserstoffsuspensionen zu messen, wobei die Stoffdichtesensoren auf ein und demselben Messprinzip basieren, dass Mittel vorgesehen sind, die eingerichtet sind, zur Aufstellung wenigstens einer Massenbilanz zumindest einen Teil der in eine Bilanzhülle eintretenden und aus dieser austretenden Volumen- und / oder Mengenströme zu messen, und dass weitere Mittel vorgesehen sind, die eingerichtet sind, diese Stoffdichtesensoren online und automatisch zu kalibrieren, indem diese weiteren Mittel ein Bilanzierungsmodell durch mathematische Verknüpfung aufstellen, bei der die wenigstens eine Massenbilanz und die Sensorkennlinien der Stoffdichtesensoren Berücksichtigung finden, um aus den erhaltenen Stoffdichtemesswerten die tatsächlichen Stoffdichtewerte zu ermitteln.

Bevorzugte Ausführungsformen der erfindungsgemäßen Vorrichtung sind in den Unteransprüchen angegeben.

Die Erfindung wird im folgenden anhand von Ausführungsbeispielen unter Bezugnahme auf die Zeichnung näher erläutert; in dieser zeigen:

Fig. 1    eine schematische Darstellung einer Anlage zur Herstellung einer Faserstoffbahn mit einer beispielhaften Ausführungsform der erfindungsgemäßen automatischen Kalibrierung von der Messung von Faserstoffsuspensionen dienenden Stoffdichtesensoren durch eine Online-Prozeßsimulation,

Fig. 2    eine schematische Darstellung einer linearen Sensorkennlinie,

Fig. 3    eine schematische Darstellung zweier Sensorkennlinien für zwei unterschiedliche Stoffsorten,

Fig. 4    eine schematische Darstellung einer Anlage zur Herstellung einer Faserstoffbahn mit einer beispielhaften Ausführungsform der erfindungsgemäßen automatischen Kalibrierung von Stoffdichtesensoren, bei der die Stoffdichtesensoren jeweils einer Dickstoff führenden Leitung zugeordnet sind, und

Fig. 5    eine schematische Darstellung einer Anlage zur Herstellung einer Faserstoffbahn mit einer beispielhaften Ausführungsform der erfindungsgemäßen automatischen Kalibrierung von Stoffdichtesensoren, bei der u.a. die Lippenmenge gemessen und eine Massenbilanz aufgestellt wird, in die insbesondere der Trockengehalt der Faserstoffbahn nach der Presse mit einbezogen wird.

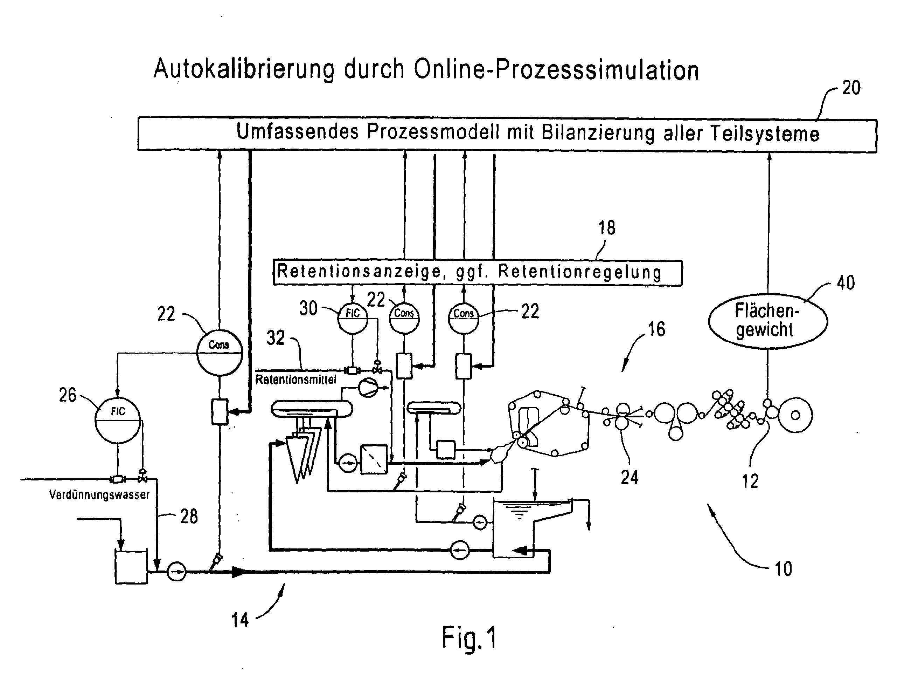

Fig. 1 zeigt in schematischer Darstellung eine beispielhafte Ausführungsform einer Anlage 10 zur Herstellung einer Faserstoffbahn 12, bei der es sich insbesondere um eine Papier- oder Kartonbahn handeln kann. Die Anlage 10 ist mit einer beispielhaften Ausführungsform einer erfindungsgemäßen automatischen Kalibrierung von der Messung von Faserstoffsuspensionen dienenden Stoffdichtesensoren 22 durch eine Online-Prozeßsimulation ausgestattet.

Die Anlage 10 umfaßt einen konstanten Teil 14, eine Papiermaschine 16 und eine Retentionsanzeige und/ oder Retentionsregelung 18. Erfindungsgemäß ist mit Hilfe entsprechender Modellierungs- und Simulationsmittel auf einem Rechner oder dergleichen ein übergeordnetes umfassendes Prozeßmodell 20 mit einer Bilanzierung aller Teilsysteme implementiert. Dabei liefern an verschiedenen Stellen im konstanten Teil 14 vorgesehene Stoffdichtesensoren 22 entsprechende Stoffdichtemeßwerte an das übergeordnete umfassende Prozeßmodell 20. Wie anhand der Fig. 1 zu erkennen ist, kann ein Teil dieser Stoffdichtesensoren 22 den jeweiligen Meßwert gleichzeitig auch an die Retentionsanzeige bzw. -regelung 18 liefern. Beim dargestellten Ausführungsbeispiel kann mittels einer entsprechenden Meßeinrichtung 40 insbesondere auch das Flächengewicht (basis weight) oder der Trockengehalt der Papierbahn nach der Presse 24 gemessen und der betreffende Meßwert an das umfassende Prozeßmodell 20 geliefert werden.

Ein Durchflußregler 26 dient der Einstellung des Verdünnungswasserdurchflusses in einer Leitung 28, und ein Durchflußregler 30 dient der Einstellung eines Retentionsmitteldurchflusses in einer Leitung 32.

Die im konstanten Teil 14 vorgesehenen Stoffdichtesensoren 22 dienen der Messung der Konzentrationen von Faserstoffsuspensionen, d.h. sowohl von Suspensionen mit hoher Stoffdichte (Konzentration g/l von Faserstoff) als auch von Suspensionen mit kleiner Stoffdichte (z.B. Siebwasser I, Siebwasser II).

Zumindest ein Teil der insbesondere im konstanten Teil 14 der Anlage 10 vorgesehenen Stoffdichtesensoren 22 werden nun online und automatisch kalibriert. Dazu werden Stoffdichtesensoren 22 verwendet, denen dasselbe Meßprinzip zugrundeliegt. Aus den mit diesen Stoffdichtesensoren 22 gemessenen Stoffdichten werden dann mit Hilfe einer Massenbilanz und den Sensorkennlinien die tatsächlichen Stoffdichtewerte ermittelt.

Vorzugsweise ist ein Stoffdichtesensor, z.B. die Meßeinrichtung 40, vorgesehen, der einen mit dem betreffenden tatsächlichen Stoffdichtewert übereinstimmenden Stoffdichtemeßwert liefert. Dieser direkt verwendbare Stoffdichtemeßwert kann dann bei der Aufstellung der Massenbilanz mit herangezogen werden, um rekursiv die betreffenden Stoffdichtesensoren 22 zu kalibrieren.

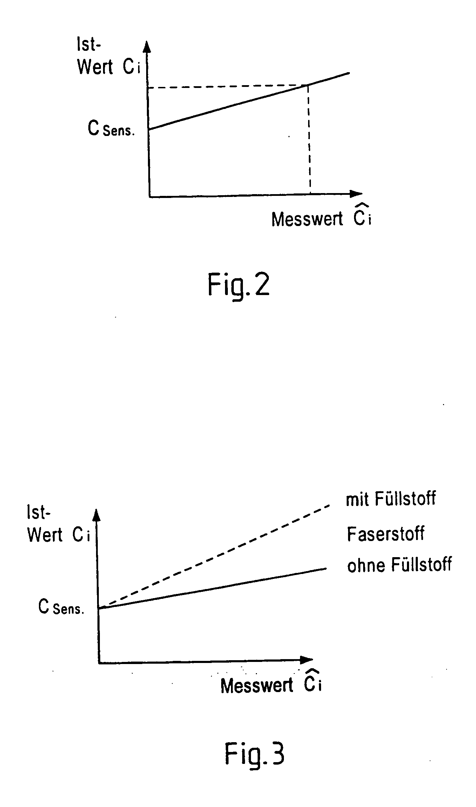

Fig. 2 zeigt in schematischer Darstellung eine typische lineare Sensorkennlinie. In dem betreffenden Diagramm sind die tatsächlichen Stoffdichtewert oder Ist-Werte $c_i$ über den Stoffdichtemeßwerten $\hat{c}_i$ dargestellt.

Das Kennlinien-Diagramm beschreibt also in der Regel einen linearen Zusammenhang zwischen den Stoffdichtemeßwerten $\hat{c}_i$ und den tatsächlichen Stoffdichtewerten $c_i$ (Ist-Werte).

In diesem Fall läßt sich eine betreffende Sensorkennlinie also wie folgt darstellen:

$$c_i = c_{Sens} + f_i \cdot \hat{c}_i \text{ (Sensorkennlinie)},$$

wobei $c_{Sens}$ konstant und bekannt ist, und $f_i$ ein von der Stoffart und vom jeweiligen Sensortyp abhängiger Proportionalitätsfaktor ist. Daher müssen also die von der erfindungsgemäßen automatischen Online-Kalibrierung betroffenen Stoffdichtesensoren 22 vom gleichen Sensortyp sein.

Fig. 3 zeigt in schematischer Darstellung zwei Sensorkennlinien für zwei unterschiedliche Stoffsorten. Dabei gilt die durchgezogene Kennlinie für Faserstoff ohne Füllstoff und die gestrichelt dargestellte Kennlinie für Faserstoff mit Füllstoff.

Überdies kann, wie bereits erwähnt, ein Stoffdichtesensor verwendet werden, der einen mit dem betreffenden tatsächlichen Stoffdichtewert übereinstimmenden Stoffdichtemeßwert liefert, wobei dieser direkt verwendbare Stoffdichtemeßwert bei der Aufstellung der Massenbilanz mit herangezogen wird.

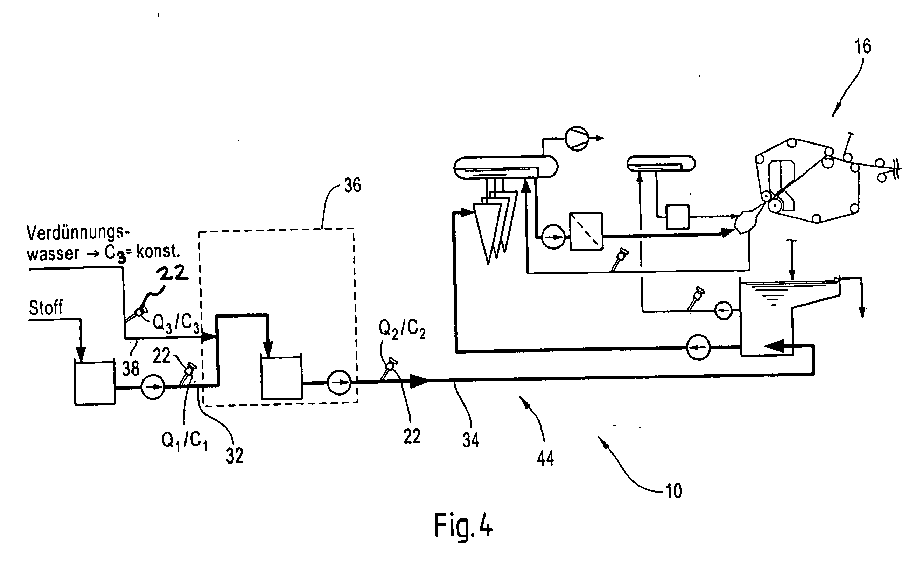

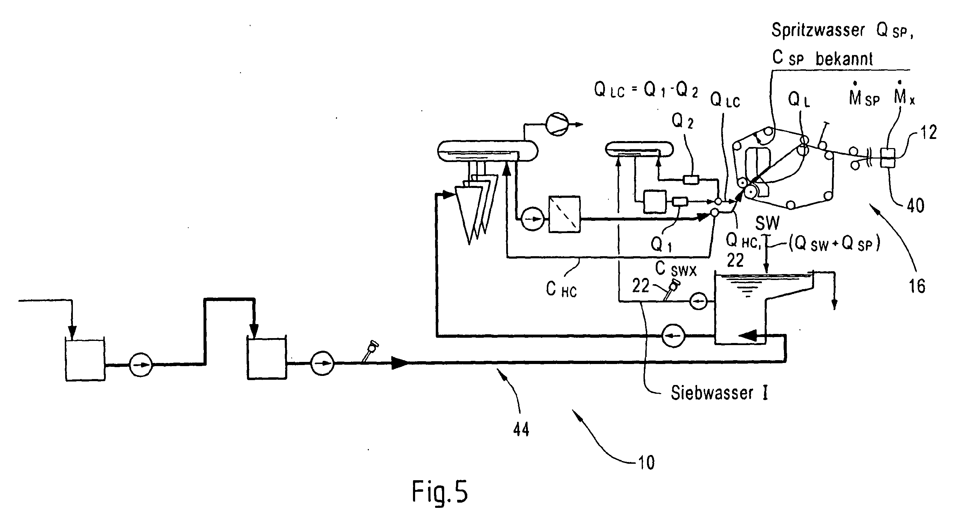

Bei den im folgenden näher beschriebenen Ausführungsbeispielen gemäß den Fig. 4 und 5 ist mittels der betreffenden Algorithmen jeweils eine Kalibrierung zweier Stoffdichtesensoren 22 über die Steigung der Sensorkennlinien und die betreffenden Massenbilanzen möglich. Werden mehr als zwei Stoffdichtesensoren 22 in die Berechnung einbezogen, können zusätzliche Kalibrierparameter ermittelt werden. So können beispielsweise bei drei Stoffdichtesensoren die beiden Variablen "a" und "b" einer Sensorkennlinie vom Typ y = a + b x bestimmt werden, und beispielsweise bei vier Sensoren die Variablen "a", "b" und "c" einer Sensorkennlinie vom Typ $y = a + b x + c x^2$, usw.

Fig. 4 zeigt in schematischer Darstellung eine Anlage 10 zur Herstellung einer Faserstoffbahn mit einer beispielhaften Ausführungsform der erfindungsgemäßen automatischen Kalibrierung von Stoffdichtesensoren 22, bei der die Stoffdichtesensoren 22 jeweils einer Dickstoff führenden Leitung 32 bzw. 34 zugeordnet sind. Es wird eine Massenbilanz für eine Bilanzhülle bzw. einen entsprechenden Teilabschnitt 36 der Herstellungsanlage 10 aufgestellt, dem über die Leitung 32 Dickstoff und über eine Leitung 38 Verdünnungswasser zugeführt und aus dem über die Leitung 34 verdünnter Dickstoff abgeführt wird. Die beiden zu kalibrierenden Stoffdichtesensoren sind in der Leitung 32 bzw. in der Leitung 34 angeordnet, die jeweils Dickstoff (unverdünnt bzw. verdünnt) führen.

Bei dieser Dickstoffkalibrierung wird vorausgesetzt, daß alle Volumen- oder Mengenstrommessungen $Q_i$ exakt sind. So können beispielsweise der Mengenstrom $Q_1$ in der Dickstoff führenden Leitung 32 und der Mengenstrom $Q_3$ in der Verdünnungswasser führenden Leitung 38 gemessen und daraus anhand der Beziehung $Q_2 = Q_1 + Q_3$ der Mengenstrom $Q_2$ in der verdünnten Dickstoff führenden Leitung 34 bestimmt werden.

Bei den folgenden Betrachtungen gilt:

$Q_i$ Mengenstrom [1/min], als exakt angenommen
$\hat{c}_i$ Stoffdichte [%], zu kalibrierender Meßwert
$c_i$ exakte Stoffdichte, bestimmt aus dem Meßwert $\hat{c}_i$ nach der Kalibrierung.

Mit der Durchführung der Massenbilanz um die Bilanzhülle 36 ergibt sich die folgende Beziehung:

$$Q_1 \cdot c_1 + Q_3 \cdot c_3 = (Q_1 + Q_3) \cdot C_2,$$

wobei $c_3$ bekannt ist oder aus dem in der Fig. 5 dargestellten weiteren Teil interaktiv bestimmt werden kann.

Durch eine entsprechende Umformung ergibt sich:

$$c_1 = \frac{Q_1 + Q_3}{Q_1} \cdot c_2 - \frac{Q_3}{Q_1} \cdot c_3,$$

wobei die Multiplikanden vor "$c_2$" und "$c_3$" berechenbare Faktoren bzw. Konstanten sind.

Daraus ergibt sich die folgende Beziehung:

$$(1) \qquad c_1 = \text{Konst.} + f \cdot c_2$$

Damit ist die exakte Beziehung zwischen den beiden Stoffdichten $c_1$ und $c_2$ angegeben, wobei diese jedoch nicht exakt gemessen werden. Die Stoffdichten $c_1$ und $c_2$ sind vielmehr durch Kalibrieren der erhaltenen Meßwerte $\hat{c}_i$ und $\hat{c}_2$ zu bestimmen.

Die bekannte Sensorkennlinie lautet wie folgt:

$$c_i = c_{Sens} + f_i \cdot \hat{c}_i,$$

wobei in dieser bekannten Sensorkennlinie "$c_{Sens}$" eine Konstante und bekannt ist und "$f_i$" stoffabhängig variabel ist.

Es ergibt sich somit:

$$c_1 = c_{Sens} + f_x \cdot \hat{c}_1$$

$$c_2 = c_{Sens} + f_x \cdot \hat{c}_2$$

Da in beiden zu kalibrierenden Stoffdichtesensoren derselbe Stoff fließt und Sensoren vom gleichen Typ verwendet werden, muß "$f_x$" ebenfalls gleich sein.

Es ergibt sich somit die folgende Beziehung:

$$(2) \qquad \frac{c_1 - c_{Sens}}{\hat{c}_1} = \frac{c_2 - c_{Sens}}{\hat{c}_2},$$

wobei $\hat{c}_1$ und $\hat{c}_2$ Stoffdichtemeßwerte und daher bekannt sind.

Mit den beiden Beziehungen (1) und (2) erhält man somit zwei Gleichungen mit zwei Unbekannten, nämlich den beiden zu bestimmenden Stoffdichten $c_1$ und $c_2$. Diese beiden Stoffdichten $c_1$ und $c_2$ können damit entsprechend bestimmt werden.

Es handelt sich im vorliegenden Fall also um eine Dickstoffkalibrierung, wobei vorausgesetzt wird, daß alle Volumenstrom- oder Mengenstrommessungen Qi exakt sind. Werden beispielsweise die Mengenströme $Q_1$ und $Q_3$ gemessen, so kann aus der Beziehung

$$Q_2 = Q_1 + Q_3$$

der Mengenstrom $Q_2$ bestimmt werden. Mit dem Aufstellen einer Massenbilanz um die Bilanzhülle 36 ergibt sich dann die obige Beziehung (1). Zusammen mit der Sensorkennlinie erhält man ein Gleichungssystem mit zwei Unbekannten. Aus der Lösung erhält man dann die tatsächlichen Werte für die Konzentrationen $c_1$ und $c_2$.

Hierbei ist es wichtig, daß die Stoffdichtesensoren in Leitungen 32, 34 eingebracht sind, in denen Faserstoff bzw. Stoff mit derselben Stoffcharakteristik fließt. Erst dadurch wird es möglich, die tatsächlichen Stoffdichtewerte mit hoher Genauigkeit zu bestimmen, da dann der Proportionalitätsfaktor "$f_i$" aller Stoffdichtesensoren gleich ist und das Gleichungssystem gelöst werden kann.

Fig. 5 zeigt in schematischer Darstellung eine Anlage 10 zur Herstellung einer Faserstoffbahn mit einer anderen beispielhaften Ausführungsform der erfindungsgemäßen automatischen Kalibrierung von Stoffdichtesensoren 22, bei der u.a. die Lippenmenge $Q_L$ gemessen und eine Massenbilanz aufgestellt wird, in die insbesondere der beispielsweise mittels der Meßeinrichtung 40, z.B. mittels eines Scanners, gemessene Trockengehalt der Faserstoffbahn 12 nach der Presse mit einbezogen wird.

Dabei wird u.a. die aus dem Stoffauflauf austretende Lippenmenge $Q_L$ (1/min) gemessen und berechnet, was als solches bereits bekannt ist.

Der im folgenden verwendete Begriff "otro" bedeutet "ofentrocken", was bedeutet, daß im Papier so gut wie kein Wasser mehr vorhanden ist. Mit "otro-Masse" wird daher die reine Feststoffmasse des Papiers angegeben.

Im übrigen gelten die folgenden Definitionen:

| | |
|---|---|
| $\dot{M}_s$: | Massenstrom aus dem Stoffauflauf auf das Formersieb |
| $Q_2$: | Siebwasser-Volumenstrom, der nicht durch den Stoffauflauf strömt, sondern rezirkuliert wird |
| $Q_1$: | Siebwasser-Volumenstrom zum Stoffauflauf |
| $(Q_1-Q_2)$: | Siebwasser-Volumenstrom durch den Stoffauflauf |
| $Q_{HC}$: | Volumenstrom an Faserstoff mit hoher Stoffdichte durch den Stoffauflauf |
| $Cswx$: | zu kalibrierende Stoffdichte des Siebwassers, hier z.B. des Siebwassers I |
| $M_x$: | Faserstoffmassenstrom (Flächengewicht) am Ende der Papiermaschine ("maschinentrocken", d.h. es ist eine nennenswerte Feuchtigkeit im Papier (1-10%)) |
| $\dot{M}_R$: | Randstreifenmassenstrom, der nach dem Former weggeführt wird |
| $\dot{M}_P$: | Massenstrom des Papiers nach der Entwässerung und vor dem Wegführen der Randstreifenmasse |
| $\dot{M}_{SW}$: | Massenstrom, der während der Entwässerung durch das Sieb hindurchtritt (Siebdurchfall) |
| $Q_P$: | Volumenstrom der Papierbahn nach der Presse |
| $C_P$: | Trockengehalt der Papierbahn nach der Presse, der gemessen werden muß |

Die Lippenmenge $Q_L$ ist eine Funktion der Strahlgeschwindigkeit und der Blendenöffnung, und sie ist bekannt. Es gilt also:

$Q_L$ = f (Strahlgeschwindigkeit, Blendenöffnung)

Für den Volumenstrom $Q_{HC}$ des Faserstoffes mit hoher Stoffdichte durch den Stoffauflauf gilt:

$$Q_{HC} = \dot{Q}_L - Q_1 + Q_2 \, ,$$

wobei die Annahme gilt, daß die Volumenmessungen exakt, d.h. nicht komponentenabhängig, sind.

Für den otro-Massenstrom auf das Sieb gilt die folgende Beziehung:

$$(1') \quad \dot{M}_S = (Q_1 - Q_2) \cdot c_{swx} + Q_{HC} \cdot C_{HC}$$

Darüber hinaus gilt:

$\dot{M}_x$ = f (Papiermaschinen-Geschwindigkeit, Bahnbreite, Feuchte),

wobei es sich hier um den otro-Massenstrom am Ende der Papiermaschine handelt, der als exakt angenommen wird.

$$\dot{M}_P \;=\; \dot{M}_X + \dot{M}_R \,,$$

wobei, wie bereits erwähnt, mit $\dot{M}_R$ der Randstreifenmassenstrom und mit $\dot{M}_P$ der otro-Blattmassenstrom auf das Sieb abzüglich des Siebdurchfalls angegeben ist.

$$\dot{M}_{SW} = \dot{M}_S - \dot{M}_P \;,$$

wobei es sich hier um den Siebdurchfall handelt.

$$Q_P = \frac{\dot{M}_P \cdot 100}{C_P} \;,$$

wobei $Q_P$ den Volumenstrom nach der Presse einschließlich des Randstreifenvolumenstroms angibt und $C_P$ bekannt ist.

$$Q_{SW} = Q_L - Q_P,$$

womit der Siebdurchfall (volumenmäßig) gemeint ist.

$$c_{SWX} = \frac{\dot{M}_{SW} \cdot 100 + \dot{M}_{SP} \cdot 100}{Q_{SW} + Q_{SP}} \;,$$

$$c_{SWX} = \frac{(\dot{M}_S - \dot{M}_P) \cdot 100 - \dot{M}_{SP} \cdot 100}{Q_{SW} + Q_{SP}} \;,$$

wobei mit $Q_{SP}$ ein Spritzwasservolumenstrom angegeben ist.

Setzt man nun in die beiden letzten Gleichungen die Größe "$\dot{M}_S$" aus der Beziehung (1') ein, so erhält man die Beziehung:

$$(2')\; c_{SWX} = \frac{100}{Q_{SW} + Q_{SP}} \Big[ (Q_1 - Q_2) \cdot c_{SWX} + Q_{HC} \cdot c_{HC} - \dot{M}_X - \dot{M}_P - \dot{M}_{SP} \Big],$$

womit eine Beziehung zwischen den zu kalibrierenden Stoffdichten cswx und CHC hergestellt ist.

Aus den Sensorkennlinien ergibt sich:

$$c_{SWX} = c_{Sens} + f_x \cdot \hat{c}_{SWX} \,,$$

$$c_{HC} = c_{Sens} + f_x \cdot \hat{c}_{HC} \,,$$

wobei $c_{Sens}$ konstant und $f_x$ eine Kalibrierkonstante ist.

Es sei angenommen, daß "$c_{Sens}$" nicht von der Stoffart abhängig ist, d.h. $c_{Sense} \neq f$ (Stoffart). Falls auch eine Abhängigkeit von der Stoffart vorliegt, d.h. $c_{Sense} = f$ (Sensor, Stoffart) gilt, ist ein zusätzlicher Sensor erforderlich.

Damit ergibt sich die Beziehung:

$$(3') \quad \frac{c_{SWX} - c_{Sens}}{\hat{c}_{SWX}} = \frac{c_{HC} - c_{Sens}}{\hat{c}_{HC}}$$

Mit den beiden Beziehungen (2') und (3') erhält man somit zwei Gleichungen mit den beiden Unbekannten $c_{swx}$ und $c_{HC}$. Die betreffenden Stoffdichten lassen sich damit genau bestimmen.

Bei der bisher üblichen Stoffdichtebestimmung über die Beziehung $c_{Swx} = c_{Sens} + f_x \cdot \hat{c}_{swx}$ war $f_x$ stark abhängig von der Stoffart. Mit der erfindungsgemäßen Lösung fällt diese Abhängigkeit nunmehr weg.

Auch bei der zuletzt beschriebenen Ausführungsform der erfindungsgemäßen Kalibrierung wird also ein Gleichungssystem aus einer Massenbilanz und den Sensorkennlinien bzw. Kalibrierungskurven aufgestellt. Wesentlich und notwendig ist dabei eine Messung (durch eine entsprechende Meßeinrichtung) der Flächenmasse nach der Papiermaschine 16 und/oder in der Trockenpartie und/oder nach der Presse, und eine Messung des Trockengehalts nach der Presse.

Wie anhand der Fig. 5 zu erkennen ist, kann am Ende der Papiermaschine 16 z.B. ein Scanner 40 vorgesehen sein, um die betreffenden Meßwerte (z.B. $\dot{M}_x$) zu erhalten.

Bei den beiden zuletzt beschriebenen Ausführungsbeispielen (vgl. die Figuren 4 und 5) ist ebenso wie beim Ausführungsbeispiel gemäß der Figur 1 jeweils eine Autokalibrierung durch eine Online-Prozeßsimulation möglich.

**Bezugszeichenliste**

| | |
|---|---|
| 10 | Herstellungsanlage |
| 12 | Faserstoffbahn |
| 14 | konstanter Teil, Stoffzuführsystem |
| 16 | Papiermaschine |
| 18 | Retentionsanzeige und/oder -regelung |
| 20 | Prozeßmodell |
| 22 | Stoffdichtesensor |
| 24 | Presse |
| 26 | Durchflußregler |
| 28 | Leitung |
| 30 | Durchflußregler |
| 32 | Leitung |
| 34 | Leitung |
| 36 | Bilanzhülle, Teilabschnitt |
| 38 | Leitung |
| 40 | Scanner |

**Patentansprüche**

1. Verfahren zum Kalibrieren von Stoffdichtesensoren (22), die bei der Herstellung einer Faserstoffbahn (12), insbesondere Papier- oder Kartonbahn, dazu dienen, für den Herstellungsprozess benötigte Stoffdichten von Faserstoffsuspensionen zu messen, wobei auf ein und demselben Messprinzip basierende Stoffdichtesensoren (22) verwendet und diese Stoffdichtesensoren (22) online und automatisch kalibriert werden, indem zur Aufstellung wenigstens einer Massenbilanz zumindest ein Teil der in eine Bilanzhülle eintretenden und aus dieser austretenden Volumen- und / oder Mengenströme gemessen werden und indem ein Bilanzierungsmodell durch mathematische Verknüpfung aufgestellt wird, bei der die wenigstens eine Massenbilanz und die Sensorkennlinien der Stoffdichtesensoren Berücksichtigung finden, um aus den erhaltenen Stoffdichtemesswerten die tatsächlichen Stoffdichtewerte zu ermitteln.

2. Verfahren nach Anspruch 1,
   **dadurch gekennzeichnet,**
   **daß** überdies ein genau arbeitender Stoffdichtesensor (40) als Kalibriersensor verwendet wird, der einen mit dem betreffenden tatsächlichen Stoffdichtewert übereinstimmenden Stoffdichtemeßwert liefert, und daß dieser direkt verwendbare Stoffdichtemeßwert bei der Aufstellung der Massenbilanz mit herangezogen wird.

3. Verfahren nach einem der vorhergehenden Ansprüche,
   **dadurch gekennzeichnet,**
   **daß** die zu kalibrierenden Stoffdichtesensoren (22) im konstanten Teil (14) der Herstellungsanlage (10) eingesetzt sind.

4. Verfahren nach einem der vorhergehenden Ansprüche,
   **dadurch gekennzeichnet,**
   **daß** die zu kalibrierenden Stoffdichtesensoren (22) lineare Sensorkennlinien besitzen.

5. Verfahren nach einem der vorhergehenden Ansprüche,
   **dadurch gekennzeichnet,**
   **daß** die zu kalibrierenden Stoffdichtesensoren (22) Leitungen (32, 34) zugeordnet sind, in denen Faserstoff mit derselben Stoffcharakteristik bzw. Stoff mit derselben Stoffcharakteristik fließt.

6. Verfahren nach einem der vorhergehenden Ansprüche,
   **dadurch gekennzeichnet,**
   **daß** die zu kalibrierenden Stoffdichtesensoren (22) jeweils einer Dickstoff führenden Leitung (32, 34) zugeordnet sind.

7. Verfahren nach einem der vorhergehenden Ansprüche,
   **dadurch gekennzeichnet,**
   **daß** eine Massenbilanz für einen Teilabschnitt (36) der Herstellungsanlage (10) aufgestellt wird, dem Dickstoff und Verdünnungswasser zugeführt und aus dem verdünnter Dickstoff abgeführt wird, wobei die zu kalibrierenden Stoffdichtesensoren (22) zur Messung der Stoffdichten des zugeführten unverdünnten Dickstoffes und des abgeführten verdünnten Dickstoffes vorgesehen sind.

8. Verfahren nach einem der vorhergehenden Ansprüche,
   **dadurch gekennzeichnet,**
   **daß** eine Massenbilanz aufgestellt wird, in die die gemessene Flächenmasse nach der Papiermaschine (16) und/oder in der Trockenpartie und/oder nach der Presse (24) und/oder der gemessene Trockengehalt nach der Presse (24) mit einbezogen wird.

9. Vorrichtung zum Kalibrieren von Stoffdichtesensoren (22), die bei der Herstellung einer Faserstoffbahn (12), insbesondere Papier- oder Kartonbahn, dazu dienen, für den Herstellungsprozess benötigte Stoffdichten von Faserstoffsuspensionen zu messen, wobei die Stoffdichtesensoren (22) auf ein und demselben Messprinzip basieren, dass Mittel vorgesehen sind, die eingerichtet sind, zur Aufstellung wenigstens einer Massenbilanz zumindest einen Teil der in eine Bilanzhülle eintretenden und aus dieser austretenden Volumen- und / oder Mengenströme zu messen, und dass weitere Mittel (20) vorgesehen sind, die eingerichtet sind, diese Stoffdichtesensoren (22) online und automatisch zu kalibrieren, indem diese weiteren Mittel (20) ein Bilanzierungsmodell durch mathematische Verknüpfung auf stellen, bei der die wenigstens eine Massenbilanz und die Sensorkennlinien der Stoffdichtesensoren (22) Berücksichtigung finden, um aus den erhaltenen Stoffdichtemesswerten die tatsächlichen Stoffdichtewerte zu ermitteln.

**10.** Vorrichtung nach Anspruch 9,
**dadurch gekennzeichnet,**
**daß** überdies ein genau arbeitender Stoffdichtesensor (40) als Kalibriersensor vorgesehen ist, der einen mit dem betreffenden tatsächlichen Stoffdichtewert übereinstimmenden Stoffdichtemeßwert liefert, und daß dieser direkt verwendbare Stoffdichtemeßwert bei der Aufstellung der Massenbilanz mit heranziehbar ist.

**11.** Vorrichtung nach einem der Ansprüche 9 oder 10,
**dadurch gekennzeichnet,**
**daß** die zu kalibrierenden Stoffdichtesensoren (22) lineare Sensorkennlinien besitzen.

**12.** Vorrichtung nach einem der Ansprüche 9-11,
**dadurch gekennzeichnet,**
**daß** die Mittel (20) eingerichtet sind, um eine Massenbilanz für einen Teilabschnitt der Herstellungsanlage (10) aufzustellen, dem Dickstoff und Verdünnungswasser zugeführt und aus dem verdünnter Dickstoff abgeführt wird, wobei die zu kalibrierenden Stoffdichtesensoren (22) zur Messung der Stoffdichten des zugeführten unverdünnten Dickstoffes und des abgeführten verdünnten Dickstoffes vorgesehen sind.

**13.** Vorrichtung nach einem der Ansprüche 9-12,
**dadurch gekennzeichnet,**
**daß** Mittel (40) vorgesehen sind, die eingerichtet sind, um die Flächenmasse nach der Papiermaschine (16) und/ oder in der Trockenpartie und/oder nach der Presse (24) und/oder den Trockengehalt nach der Presse (24) zu messen, und daß die Mittel (20) eingerichtet sind, um eine Massenbilanz aufzustellen, in die zumindest einer der genannten Meßwerte mit einbezogen ist.

**14.** Maschine zur Herstellung einer Faserstoffbahn mit einer Vorrichtung zum Kalibrieren von Stoffdichtesensoren (22) nach einem der Ansprüche 9 oder 10, bei der die zu kalibrierenden Stoffdichtesensoren (22) im konstanten Teil (14) der Herstellungsanlage (10) vorgesehen sind.

**15.** Maschine nach Anspruch 14,
**dadurch gekennzeichnet,**
**daß** die zu kalibrierenden Stoffdichtesensoren (22) Leitungen (32, 34) zugeordnet sind, in denen Faserstoff mit derselben Stoffcharakteristik bzw. Stoff mit derselben Stoffcharakteristik fließt.

**16.** Maschine nach Anspruch 14 oder 15,
**dadurch gekennzeichnet,**
**daß** die zu kalibrierenden Stoffdichtesensoren (22) jeweils einer Dickstoff führenden Leitung (32, 34) zugeordnet sind.

**Claims**

**1.** Method for calibrating consistency sensors (22) which, during the production of a fibrous web (12), in particular a paper or paperboard web, serve to measure the consistencies of fibrous stock suspensions which are required for the production process, consistency sensors (22) being used which are based on one and the same measuring principle and the said consistency sensors (22) being calibrated online and automatically by at least one part of the volumetric and/or mass flows which enter into and emerge from a balance sleeve being measured in order to produce at least one mass balance and by a balancing model being produced by mathematic combination, in which balancing model the at least one mass balance and the sensor characteristic curves of the consistency sensors are taken into consideration, in order to determine the actual consistency values from the obtained consistency measured values.

**2.** Method according to Claim 1, **characterized in that**, moreover, a precisely operating consistency sensor (40) is used as calibration sensor which supplies a consistency measured value which corresponds to the relevant actual consistency value, and **in that** the said consistency measured value which can be used directly is also used in the production of the mass balance.

**3.** Method according to one of the preceding claims, **characterized in that** the consistency sensors (22) which are to be calibrated are used in the constant part (14) of the production plant (10).

4. Method according to one of the preceding claims, **characterized in that** the consistency sensors (22) which are to be calibrated have linear sensor characteristic curves.

5. Method according to one of the preceding claims, **characterized in that** the consistency sensors (22) which are to be calibrated are assigned to lines (32, 34), in which fibrous stock having the same stock characteristics or stock having the same stock characteristics flows.

6. Method according to one of the preceding claims, **characterized in that** the consistency sensors (22) which are to be calibrated are assigned in each case to a line (32, 34) which carries thick stock.

7. Method according to one of the preceding claims, **characterized in that** a mass balance is produced for a part section (36) of the production plant (10), which part section (36) is supplied with thick stock and dilution water and from which part section (36) diluted thick stock is removed, the consistency sensors (22) which are to be calibrated being provided for measuring the consistencies of the supplied undiluted thick stock and the removed diluted thick stock.

8. Method according to one of the preceding claims, **characterized in that** a mass balance is produced, in which the measured mass per unit area after the papermaking machine (16) and/or in the drying section and/or after the press (24) and/or the measured dryness after the press (24) are/is included.

9. Apparatus for calibrating consistency sensors (22) which, during the production of a fibrous web (12), in particular a paper or paperboard web, serve to measure the consistencies of fibrous stock suspensions which are required for the production process, the consistency sensors (22) being based on one and the same measuring principle, in that means are provided which are set up, for producing at least one mass balance, to measure at least one part of the volumetric and/or mass flows which enter into and emerge from a balance sleeve, and in that further means (20) are provided which are set up to calibrate the said consistency sensors (22) online and automatically by the said further means (20) producing a balancing model by mathematic combination, in which balancing model the at least one mass balance and the sensor characteristic curves of the consistency sensors (22) are taken into consideration, in order to determine the actual consistency values from the obtained consistency measured values.

10. Apparatus according to Claim 9, **characterized in that**, moreover, a precisely operating consistency sensor (40) is provided as calibration sensor which supplies a consistency measured value which corresponds to the relevant actual consistency value, and **in that** the said consistency measured value which can be used directly is also used in the production of the mass balance.

11. Apparatus according to Claim 9 or 10, **characterized in that** the consistency sensors (22) which are to be calibrated have linear sensor characteristic curves.

12. Apparatus according to one of Claims 9 to 11, **characterized in that** the means (20) are set up in order to produce a mass balance for a part section of the production plant (10), which part section is supplied with thick stock and dilution water and from which part section diluted thick stock is removed, the consistency sensors (22) which are to be calibrated being provided for measuring the consistencies of the supplied undiluted thick stock and the removed diluted thick stock.

13. Apparatus according to one of Claims 9 to 12, **characterized in that** means (40) are provided which are set up to measure the mass per unit area after the papermaking machine (16) and/or in the drying section and/or after the press (24) and/or the dryness after the press (24), and **in that** means (20) are provided in order to produce a mass balance, in which at least one of the abovementioned measured values is included.

14. Machine for producing a fibrous web having an apparatus for calibrating consistency sensors (22) according to either of Claims 9 and 10, in which the consistency sensors (22) which are to be calibrated are provided in the constant part (14) of the production plant (10).

15. Machine according to Claim 14, **characterized in that** the consistency sensors (22) which are to be calibrated are assigned to lines (32, 34), in which fibrous stock having the same stock characteristics or stock having the same stock characteristics flows.

**16.** Machine according to Claim 14 or 15, **characterized in that** the consistency sensors (22) which are to be calibrated are assigned in each case to a line (32, 34) which carries thick stock.

**Revendications**

**1.** Procédé pour calibrer des détecteurs de masse spécifique (22), qui servent à mesurer les masses spécifiques de suspensions de matières fibreuses, nécessaires lors du processus de fabrication lors de la fabrication d'une bande de matière fibreuse (12), en particulier d'une bande de papier ou de carton, dans lequel on utilise des détecteurs de masse spécifique (22) basés sur un seul et même principe de mesure et on calibre en ligne et automatiquement ces détecteurs de masse spécifique (22), par le fait que l'on mesure au moins une partie des débits volumétriques et/ou massiques entrant dans une enceinte de balance et sortant de celle-ci afin d'établir au moins un bilan massique et par le fait que l'on établit un modèle de bilan par une relation mathématique, dans laquelle on tient compte dudit au moins un bilan massique et des courbes caractéristiques de détection des détecteurs de masse spécifique, pour déterminer les valeurs de masse spécifique effectives à partir des valeurs de mesure de masse spécifique obtenues.

**2.** Procédé selon la revendication 1, **caractérisé en ce que** l'on utilise en outre un détecteur de masse spécifique (40) opérant avec précision comme détecteur de calibrage, qui fournit une valeur de mesure de masse spécifique correspondant à la valeur de masse spécifique effective concernée, et **en ce que** cette valeur de mesure de masse spécifique directement utilisable est utilisée pour l'établissement du bilan massique.

**3.** Procédé selon l'une quelconque des revendications précédentes, **caractérisé en ce que** les détecteurs de masse spécifique à calibrer (22) sont installés dans la partie constante (14) de l'installation de fabrication (10).

**4.** Procédé selon l'une quelconque des revendications précédentes, **caractérisé en ce que** les détecteurs de masse spécifique à calibrer (22) possèdent des courbes caractéristiques de détection linéaires.

**5.** Procédé selon l'une quelconque des revendications précédentes, **caractérisé en ce que** les détecteurs de masse spécifique à calibrer (22) sont associés à des conduites (32, 34), dans lesquelles il circule une matière fibreuse avec la même caractéristique de matière ou une matière avec la même caractéristique de matière.

**6.** Procédé selon l'une quelconque des revendications précédentes, **caractérisé en ce que** les détecteurs de masse spécifique à calibrer (22) sont respectivement associés à une conduite (32, 34) transportant un liquide épais.

**7.** Procédé selon l'une quelconque des revendications précédentes, **caractérisé en ce que** l'on établit un bilan massique pour une section partielle (36) de l'installation de fabrication (10), à laquelle on fournit du liquide épais et de l'eau de dilution et de laquelle on évacue un liquide épais dilué, dans lequel les détecteurs de masse spécifique à calibrer (22) sont prévus pour mesurer les masses spécifiques du liquide épais non dilué fourni et du liquide épais dilué évacué.

**8.** Procédé selon l'une quelconque des revendications précédentes, **caractérisé en ce que** l'on établit un bilan massique, dans lequel on tient compte du grammage mesuré après la machine à papier (16) et/ou dans la section de séchage et/ou après la presse (24) et/ou de la teneur en humidité mesurée après la presse (24).

**9.** Dispositif pour calibrer des détecteurs de masse spécifique (22), qui servent à mesurer les masses spécifiques de suspensions de matières fibreuses, nécessaires lors du processus de fabrication lors de la fabrication d'une bande de matière fibreuse (12), en particulier d'une bande de papier ou de carton, dans lequel des détecteurs de masse spécifique (22) sont basés sur un seul et même principe de mesure, il est prévu des moyens qui sont conçus, pour établir au moins un bilan massique, pour mesurer au moins une partie des débits volumétriques et/ou massiques entrant dans une enceinte de balance et sortant de celle-ci, et il est prévu d'autres moyens (20) qui sont conçus pour calibrer en ligne et automatiquement ces détecteurs de masse spécifique (22), par le fait que ces autres moyens (20) établissent un modèle de bilan par une relation mathématique, dans laquelle on tient compte dudit au moins un bilan massique et des courbes caractéristiques de détection des détecteurs de masse spécifique (22), pour déterminer les valeurs de masse spécifique effectives à partir des valeurs de mesure de masse spécifique obtenues.

**10.** Dispositif selon la revendication 9, **caractérisé en ce qu'**il est prévu en outre un détecteur de masse spécifique (40) opérant avec précision comme détecteur de calibrage, qui fournit une valeur de mesure de masse spécifique correspondant à la valeur de masse spécifique effective concernée, et **en ce que** cette valeur de mesure de masse spécifique directement utilisable peut être utilisée pour l'établissement du bilan massique.

**11.** Dispositif selon l'une quelconque des revendications 9 ou 10, **caractérisé en ce que** les détecteurs de masse spécifique à calibrer (22) possèdent des courbes caractéristiques de détection linéaires.

**12.** Dispositif selon l'une quelconque des revendications 9 à 11, **caractérisé en ce que** les moyens (20) sont conçus pour établir un bilan massique pour une section partielle de l'installation de fabrication (10), à laquelle on fournit du liquide épais et de l'eau de dilution et de laquelle on évacue un liquide épais dilué, dans lequel les détecteurs de masse spécifique à calibrer (22) sont prévus pour mesurer les masses spécifiques du liquide épais non dilué fourni et du liquide épais dilué évacué.

**13.** Dispositif selon l'une quelconque des revendications 9 à 12, **caractérisé en ce qu'**il est prévu des moyens (40) conçus pour mesurer le grammage après la machine à papier (16) et/ou dans la section de séchage et/ou après la presse (24) et/ou la teneur en humidité après la presse (24), et **en ce que** les moyens (20) sont conçus pour établir un bilan massique, dans lequel on tient compte d'au moins une des valeurs de mesure précitées.

**14.** Machine de fabrication d'une bande de matière fibreuse comportant un dispositif pour calibrer des détecteurs de masse spécifique (22) selon l'une quelconque des revendications 9 ou 10, dans laquelle les détecteurs de masse spécifique à calibrer (22) sont prévus dans la partie constante (14) de l'installation de fabrication (10).

**15.** Machine selon la revendication 14, **caractérisée en ce que** les détecteurs de masse spécifique à calibrer (22) sont associés à des conduites (32, 34), dans lesquelles il circule une matière fibreuse avec la même caractéristique de matière ou une matière avec la même caractéristique de matière.

**16.** Machine selon la revendication 14 ou 15, **caractérisée en ce que** les détecteurs de masse spécifique à calibrer (22) sont respectivement associés à une conduite (32, 34) transportant un liquide épais.

# Autokalibrierung durch Online-Prozesssimulation

Fig.1

Fig.2

Fig.3

Verdünnungswasser → $C_3$ = konst.

Stoff

$Q_3/C_3$

$Q_1/C_1$

$Q_2/C_2$

Fig.4

EP 1 255 103 B1

Fig.5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10043142 A1